# EUROPEAN PATENT SPECIFICATION

(11) **EP 3 658 045 B1**
(45) Date of publication and mention of the grant of the patent: **24.04.2024**
(21) Application number: 18755361.5
(22) Date of filing: 27.07.2018
(51) Int. Cl.: A61B 18/14, A61M 25/00, A61B 17/34, A61B 8/08, A61B 8/12, A61M 25/06, A61M 29/00, A61M 25/10

(54) **DIRECTIONAL BALLOON TRANSSEPTAL INSERTION DEVICE FOR MEDICAL PROCEDURES**
VORRICHTUNG ZUR TRANSSEPTALEN EINFÜHRUNG EINES GERICHTETEN BALLONS FÜR MEDIZINISCHE VERFAHREN
DISPOSITIF D'INSERTION TRANSSEPTAL À BALLONNET DIRECTIONNEL POUR PROCÉDURES MÉDICALES

(30) Priority: 28.07.2017 US 201762538552 P; 29.11.2017 US 201762592061 P
(43) Date of publication of application: 03.06.2020
(73) Proprietor: East End Medical LLC, Lewes, DE 19958 (US)
(72) Inventor: MAINI, Brijeshwar S., West Palm Beach FL 33405 (US)
(74) Representative: Patentwerk B.V.
(86) International application number: PCT/US2018/044207
(87) International publication number: WO 2019/023653

(56) References cited:
- US-A- 4 813 934
- US-A- 6 017 323
- US-A- 6 102 926
- US-A1- 2005 159 738
- US-A1- 2007 149 995
- US-A1- 2014 309 675
- US-A1- 2015 216 620
- US-A1- 2017 135 559

## Description

### RELATED APPLICATIONS

The present invention claims priority on and from U.S. Provisional Application Serial No. 62/538,552, filed July 28, 2017 and Entitled "Directional Balloon Transseptal Insertion Device for Medical Procedures" and U.S. Provisional Application Serial No. 62/592,061, filed November 29, 2017 and Entitled "Directional Balloon Transseptal Insertion Device for Medical Procedures,".

### FIELD

The present invention relates generally to cardiac catheters, and more particularly, to a transseptal insertion device which is suitable for facilitating quick and safe transseptal puncture and insertion of a catheter through a cardiac septum to provide access to the left atrium in implementation of a left atrial intervention.

### BACKGROUND

Cardiac catheterization is a medical procedure in which a long thin tube or catheter is inserted through an artery or vein into specific areas of the heart for diagnostic or therapeutic purposes. More specifically, cardiac chambers, vessels and valves may be catheterized.

Cardiac catheterization may be used in procedures such as coronary angiography and left ventricular angiography. Coronary angiography facilitates visualization of the coronary vessels and finding of potential blockages by taking X-ray images of a patient who has received a dye (contrast material) injection into a catheter previously injected in an artery. Left ventricular angiography enables examination of the left-sided heart chambers and the function of the left-sided valves of the heart, and may be combined with coronary angiography. Cardiac catheterization can also be used to measure pressures throughout the four chambers of the heart and evaluate pressure differences across the major heart valves. In further applications, cardiac catheterization can be used to estimate the cardiac output, or volume of blood pumped by the heart per minute.

Some medical procedures may require catheterization into the left atrium of the heart. For this purpose, to avoid having to place a catheter in the aorta, access to the left atrium is generally achieved by accessing the right atrium, puncturing the interatrial septum between the left and right atria of the heart, and threading the catheter through the septum and into the left atrium. Transseptal puncture must be carried out with extreme precision, as accidental puncturing of surrounding tissue may cause very serious damage to the heart. In addition, transseptal puncture may require complicated instruments which are not helpful in guaranteeing the precision of the puncture.

The use of devices available today present many challenges for doctors attempting to puncture the interatrial septum and perform cardiac catheterization. Locating the interatrial septum, properly placing the distal end of the puncturing device at the desired location of the septum, safely puncturing the interatrial septum, avoiding accidental punctures, and tracking and maneuvering the catheter post-puncture, are among the many challenges facing those performing cardiac catheterization today.

US 2014/309675 A1 discloses a transseptal insertion device which is suitable for facilitating precise and safe transseptal puncture of a cardiac interatrial septum.

Accordingly, there is an established need for a device that is suitable for facilitating quick and safe transseptal puncturing to provide access to the left atrium in implementation of a left atrial intervention.

### SUMMARY

The invention is defined by claim 1. Further embodiments of the invention are defined by the dependent claims. Methods as such are not claimed.

Embodiments described herein overcome the disadvantages of the prior art. Embodiments provide for a device that is suitable for facilitating quick and safe transseptal puncturing to provide access to the left atrium in implementation of a left atrial intervention.

These and other advantages may be provided, for example, by a transseptal insertion device which is suitable for facilitating precise and safe transseptal puncture of a cardiac interatrial septum. The transseptal insertion device includes a sheath that defines at least one lumen therein and has a distal end that is closest to the cardiac interatrial septum of a patient when the transseptal insertion device is in use and a proximal end that is external to the patent, a balloon that is connected to the distal end of the sheath, in which the balloon, when inflated and the transseptal insertion device is in use, overhangs and extends past the distal end of the sheath, preventing accidental puncturing of the cardiac interatrial septum and stabilizing the transseptal insertion device against fossa ovalis of the cardiac interatrial septum, and a dilator that is positioned within the at least one lumen when the transseptal insertion device is in use. The dilator has a distal end and is designed to and is capable of precisely puncturing the cardiac interatrial septum without the use of a needle or other sharp instrument.

Embodiments may also overcome the disadvantages of the prior art and provide numerous advantages by including various features, including a dilator designed to remain sub-planar to the overhanging portion of the balloon until the dilator is extended to puncture the cardiac interatrial septum, the diameter of the lumen at rest being less than the diameter of the dilator, the sheath being made from malleable material capable of accommodating larger diameter devices within the lumen, the dilator including a dilator seal that is greater in diameter than the lumen and provides a water-tight seal of the lumen while the dilator seal remains within the lumen, sheath including inflation ports communicatively coupling the lumen to the balloon so that inflation liquid may be passed through lumen into the balloon, inflating the balloon, and the dilator seal being located on the distal end of the dilator such that moving the distal end of the dilator out of the lumen so that the dilator seal is external to the distal end of the sheath un-seals the lumen and causes the inflation liquid to flow out of the balloon and through the inflation ports, deflating the balloon. The transseptal insertion device may also include an energy source, such as a radio-frequency (RF) energy source, external to the proximal end of the sheath and operatively connected to the distal end of the dilator to deliver energy to the distal end of the dilator. The dilator may be designed to and is capable of precisely puncturing the cardiac interatrial septum using the energy, such as RF energy delivered to the distal end of the dilator.

Also disclosed is an example method of precisely and safely transseptal puncturing a cardiac interatrial septum. The method may include inserting a transseptal insertion device into a right atrium of a patient's heart, inflating the balloon, in which the inflated balloon overhangs and extends past the distal end of the sheath and the dilator is sub-planar to the balloon overhang, preventing accidental puncturing of the cardiac interatrial septum, extending the distal end of the sheath so that the inflated balloon is positioned against the fossa ovalis of the cardiac interatrial septum at a desired puncture point, thereby stabilizing the transseptal insertion device against fossa ovalis, extending the distal end of the dilator past the balloon overhang, puncturing the cardiac interatrial septum with the dilator without the use of a needle or other sharp instrument. Puncturing the cardiac interatrial septum with the dilator may include applying RF energy through the dilator to the cardiac interatrial septum. Extending the distal end of the dilator past the balloon overhang may cause the balloon to deflate. The method may include extending the distal end of the sheath past the cardiac interatrial septum and reinflating the balloon.

These and other advantages may be provided, for example, by a transseptal insertion device which is suitable for facilitating precise and safe transseptal puncture of a cardiac interatrial septum. The transseptal insertion device may include a sheath that defines a lumen therein and has a distal end that is closest to the cardiac interatrial septum of a patient when the transseptal insertion device is in use and a proximal end that is external to the patent, the sheathe including inflation ports near the distal end that enable inflation fluid in the lumen to exit and enter the sheath. The device may further include a balloon that is connected to the distal end of the sheath, in which the balloon, when inflated and the transseptal insertion device is in use, overhangs and extends past the distal end of the sheath, preventing accidental puncturing of the cardiac interatrial septum and stabilizing the transseptal insertion device against fossa ovalis of the cardiac interatrial septum and the balloon is positioned over the inflation ports of the sheath so that inflation fluid may exit the sheath and enter the balloon, inflating the balloon, and exit the balloon and enter the sheath, deflating the balloon. The device may further include a dilator that is positioned within the lumen when the transseptal insertion device is in use. The dilator has a distal end and includes a dilator seal at the distal end that occludes the lumen when the dilator is sub-planar to the balloon overhang so that the balloon remains inflated after inflation fluid is passed through the lumen into the balloon and automatically deflates when the distal end of the dilator is extended sufficiently past the distal end of the sheath so that the dilator seal does not occlude the lumen.

These and other objects, features, and advantages of embodiments of the present invention will become more readily apparent from the attached drawings and the detailed description, which follow.

### Brief Description of the Drawings

The preferred embodiments described herein and illustrated by the drawings hereinafter be to illustrate and not to limit the invention, where like designations denote like elements, and in which:
FIG. 1A is a front perspective, cross-sectional view of an embodiment of a transseptal insertion device.
FIG. 1B is a front perspective, cross-sectional view of an embodiment of a transseptal insertion device showing a dilator extending partially through and extending out from device.
FIG. 1C is a front perspective, cross-sectional view of an embodiment of a transseptal insertion device showing a dilator extending partially through the device.
FIG. 2 is a front perspective, cross-sectional view of an embodiment of a transseptal insertion device showing inflated overhanging balloon and dilator positioned within device and subplanar to overhanging balloon.
FIG. 3 is a cross-sectional, end view of an embodiment of a transseptal insertion device and dilator shown prior to puncturing an interatrial cardiac septum with inflated overhanging balloon.
FIG. 4 is a front perspective, cross-sectional view of an embodiment of a transseptal insertion device with dilator advanced forward in order to tent an interatrial septum.
FIG. 5 is a front perspective, cross-sectional view of an embodiment of a transseptal insertion device with a transseptal wire advanced post-puncture through interatrial septum.
FIGS. 6A-6C are front perspective, cross-sectional views of an embodiment of a flexible transseptal insertion device with different angulations
FIG. 7 is a front perspective, cross-sectional view of an embodiment of a transseptal insertion device with radiofrequency energy capability.
FIG. 8 is a side view of an embodiment of transseptal insertion device with an overhanging balloon with marking.
FIG. 9 is a side view of an embodiment of transseptal insertion device with an overhanging balloon with a marker band.
FIG. 10 is a cross-sectional side view of an embodiment of a transseptal insertion device in accordance with the present claimed invention that includes a dilator with an electrode tip
FIG. 11A is a side view of an embodiment of a dilator that may be used in embodiments of a transseptal insertion device.
FIG. 11B is a side view of a distal end of an embodiment of a dilator that may be used in embodiments of a transseptal insertion device.
FIG. 12A and 12B are side views of an embodiment of a transseptal insertion device in accordance with the present claimed invention, and interatrial septum, that includes a dilator with an ablation tip.
FIG. 13 is a side view of an embodiment of a transseptal insertion device with mechanical deflection capability.
FIG. 14 is side views of embodiments of curved dilators that may be used in embodiments of a transseptal insertion device.
FIG. 15 is a cross-sectional, side view of an embodiment of a steerable transseptal insertion device.
FIG. 16 is a perspective side view of a proximal end of an embodiment of a transseptal insertion device showing a handle and a stabilizer.

### Detailed Description

The following detailed description is merely exemplary in nature and is not intended to limit the described embodiments or the application and uses of the described embodiments. As used herein, the word "exemplary" or "illustrative" means "serving as an example, instance, or illustration." Any implementation described herein as "exemplary or "illustrative" is not necessarily to be construed as preferred or advantageous over other implementations. All of the implementations described below are exemplary implementations provided to enable persons skilled in the art to make or use the embodiments of the disclosure and are not intended to limit the scope of the disclosure, which is defined by the claims. Furthermore, there is no intention to be bound by any expressed or implied theory presented in the preceding technical field, background, brief summary or the following detailed description. It is also to be understood that the specific devices and processes illustrated in the attached drawings, and described in the following specification, are simply exemplary embodiments of the inventive concepts defined in the appended claims. Hence, specific dimensions and other physical characteristics relating to the embodiments disclosed herein are not to be considered as limiting, unless the claims expressly state otherwise.

Embodiments of the present disclosure are directed to a transseptal insertion device or catheter which is suitable for facilitating quick and safe transseptal insertion of a needle or catheter through an interatrial cardiac septum/septal wall to provide access to the left atrium in implementation of a left atrial intervention. The transseptal insertion device is elongated yet has a relatively reduced length, and can be easily and safely turned within an atrium of the heart to achieve a correct orientation towards the cardiac septum.

In a first implementation, an embodiment includes a transseptal insertion device which is suitable for facilitating a precise and safe transseptal insertion of a needle, wire, dilator or catheter through a cardiac septum. The transseptal insertion device includes an overhanging balloon that, *e.g.*, measures 2-12 mm and is either air or fluid filled when inflated. The dilator is sub-planar to the balloon at the time of contact with the atrial tissue (*e.g*., the interatrial septum).

In operation of an embodiment, once the balloon is noted to tent the interatrial septum, the dilator is advanced to a pre-specified position which is controlled by the movement of the dilator, which results in further tenting of the septum. Once it is confirmed that the tenting of the interatrial septum is accurate, the transseptal needle may be advanced across the septum. Following this the balloon may be deflated or may be kept inflated for more stability and for purpose of preventing the sheath from advancing too deep into the left atrium.

In another embodiment, the transseptal insertion device may include multiple dilators that may be inserted into the transseptal insertion device sheath. Each dilator may be pre-configured at a different angle so that, when inserted at in the sheath, the dilator will cause the transseptal insertion device to bend or flex so that the angle of a dilator to the interatrial septum to vary from 0-270°. This allows the transseptal insertion device to target different areas of the left atrium and parts of the heart accessible through the left atrium (*e.g*., the pulmonary veins, left atrial appendage, mitral valve, or the left ventricle). Each of these different areas of parts may require a different puncture/insertion point through the septum wall and a different angle of puncture and angle of approach in the left atrium. The pre-configured dilators enable the desired angulation to be chosen and effectuated.

Another embodiment of the transseptal insertion device may include an actuator which allows flexion and extension of the catheter and the transseptal sheath, which would, therefore, allow for motion of the transseptal sheath in all three-dimensional planes (*i.e.,* x, y, z) and in both directions in each plane (*e.g.,* up and down, left and right, etc.). In embodiments, the transseptal sheath used may have a variety of diameters or gauges. For example, the French (Fr) size or gauge of the transseptal sheath may range from 6-40 Fr in embodiments. The different gauge allows different size devices to be inserted through the transseptal insertion device.

Additionally, the dilator tip may house a cap, crown, or electrode tip that is connected to a thermal, laser, sonic, electrical, or radiofrequency energy source via the dilator at an exterior hub connected to the dilator through the proximal end of the transseptal insertion device. Accordingly, the dilator tip may deliver energy to the septum to create a safe and controlled puncture. In this manner, embodiments avoid the need to use needles and the disadvantages inhered therein. As noted, the types of energy delivered could be heat, cold, laser, sonic, electrical, or radiofrequency.

In an embodiment that includes a sheath dilator capable of transmitting energy, there is no need for a sharp, metallic transseptal needle. The dilator itself therefore may be moved across the interatrial septum and the lumen of the dilator then be used to pass a variety of wires or other tools into the left atrium. The lumen of the dilator may also be used for pressure monitoring and assessment of blood oxygen saturation through various sensors deployed in the lumen. For example, various devices may be attached to the dilator through an external hub located at the proximal end of the transseptal insertion device. Such devices may include a fluid-filled pressure transducer to measure pressure, *e.g*., in the atrial chamber, a solid-state sensor for measuring pressure, oximetry, or other characteristics of the atrial chamber, and/or a device for drawing fluid (*e.g*., blood) from the atrium for testing.

In an embodiment in which the dilator carries energy, the dilator may include a wire that may be energized by the dilator. The wire may be advanced into the left atrium to deliver energy to puncture the left atrial septum. The wire may be, for example, a wire with a 0.0014 or larger diameter. Such wires would significantly decrease the footprint of the device entering the left atrium.

In an embodiment, in accordance with the present claimed invention, the sheath balloon is inflated using air or fluid via the sheath and there is no separate port or hypotube for inflation or deflation of the balloon. Such embodiments may include inflation ports in the sheath and a dilator seal on the dilator. The inflation ports may be implemented as multiple holes in the transseptal insertion device sheath that permit inflation fluid or gas to flow through a lumen in the sheath to the balloon and back out again when unsealed by the moving dilator seal

The dilator seal may be a bump or ridge located near the distal end of the dilator. When the dilator seal is covered by the overhanging sheath balloon, the seal is closed and the balloon may remain inflated. As the dilator is advanced beyond the overhanging balloon, the dilator seal moves beyond the seal zone, the dilator seal is opened or unsealed, resulting in the balloon automatically deflating. Prior art mechanisms require multiple parts on two different components that move relative to one another to seal and unseal an inflation chamber. The present embodiment requires only the ridge on the dilator.

When the balloon deflates, the inflation liquid or gas, which is inert, flows out of the balloon and into the heart, through which it is absorbed into the blood. The inflation air or fluid may include contrast agents enabling easier detection of inflation and deflation using imaging.

Embodiments may include radiopaque and echogenic markers on the balloon and the dilator. These markers may be in the form of letters, such as an E or a C. These markers allow for the appropriate positioning of the catheter in the 3-dimensional space (*e.g*., of the atrium) using imaging to view the markers and, therefore, the position of the balloon and dilator.

Embodiments may include a ring or band in the middle of the balloon. The ring in the middle of the balloon is for same purpose as the letter markings above - namely, to act as a navigational guide.

Various embodiments as described above are illustrated by the drawings. Shown throughout the drawings, embodiments of the present invention is directed toward a transseptal insertion device which is suitable for facilitating quick and safe transseptal puncturing of an interatrial septum and insertion of a catheter there thru to provide access to the left atrium in implementation of a left atrial intervention. Embodiments are suitable for other uses as well. Referring to the drawings described below, exemplary embodiments of a transseptal insertion device are illustrated. Together, the drawings illustrate an example method of using transseptal insertion device to puncture interatrial cardiac septum and extend and insert catheter (or other component inserted through transseptal insertion device) across interatrial septum and into left atrium. As shown, the transseptal insertion device is generally elongated and arranged along a longitudinal axis.

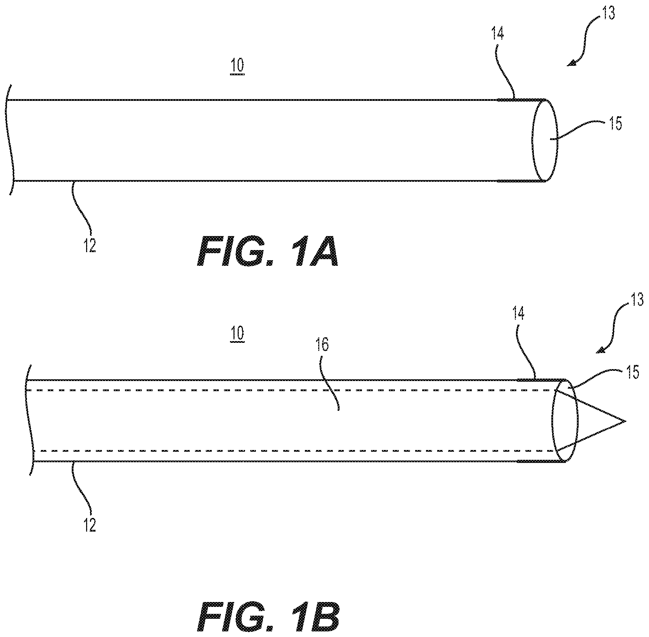

With reference now to FIGS. 1A-1C, shown is an embodiment of transseptal insertion device or catheter **10.** Shown is the distal end of transseptal insertion device **10,** *i.e.,* the end of device **10** with opening through which dilator, catheter, and needle may extend, e.g., to puncture interatrial cardiac septum. As shown in FIG. 1A, transseptal insertion device **10** includes outer sheath or balloon shaft **12** and balloon **14** located at distal tip **13** of transseptal insertion device **10.** Sheath **12** may contain and define a center lumen **15.** Sheath **12** may be fabricated from various materials, including, *e.g.*, polymers, including thermoplastics elastomers (TPEs) such as PEBA (*e.g*., Pebax^{®}), nylons, thermoplastic polyurethanes (TPUs) such as Pellathane^{®}, similar materials and combinations thereof. Sheath **12** may be referred to as catheter and used in cardiac catheterizations. After puncture, sheath **12** may be inserted through septum into left atrium. Alternatively, sheath **12** may contain a separate catheter that is inserted through septum post puncture.

Transseptal insertion device **10** also includes dilator **16,** positioned in center lumen **15,** as shown in FIG. 1B. Balloon **14** is preferably sealed, air-tight and water-tight, on both its ends to sheath **12,** with openings or one or more inflation holes (not shown) in balloon **14** that open to lumen **15.** Connected to sheath **12** as such, inflation liquid or gas may be passed through inflation holes into balloon **14,** inflating balloon **14.** Lumen **15** is kept sealed by dilator seal (not shown) that forms air and water tight seal of lumen **15** (until dilator seal on dilator **16** passes distal tip **13** and exits lumen **15**).

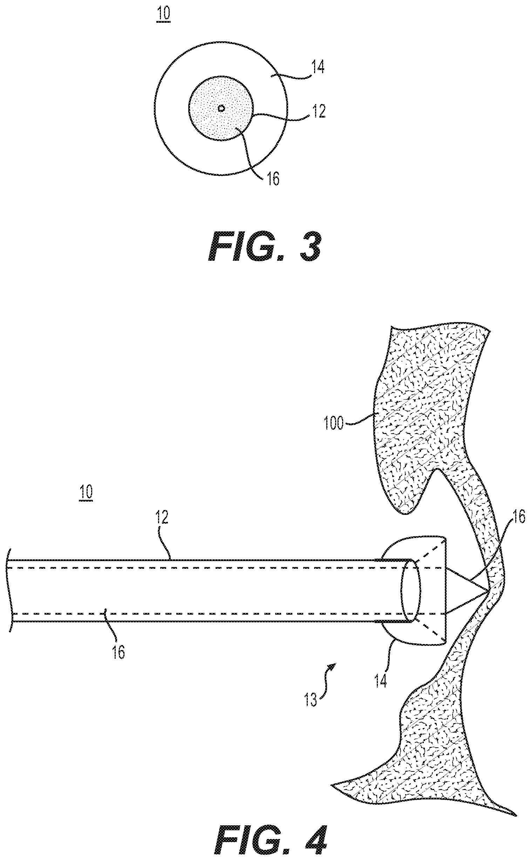

With continuing reference to FIG. 1A, in view shown, overhanging balloon **14** is uninflated. Although cross-section of balloon **14** is shown on top and bottom of distal tip **13,** balloon **14** preferably extends around circumference of distal tip or end **13** of transseptal insertion device **10** (see FIG. 4). Overhanging balloon **14** is of form such that balloon **14** overhangs or extends from distal tip **13** of sheath **12** when inflated.

In FIG. 1B, dilator **16** is shown positioned within and partially extending out of sheath **12,** past distal tip **13** of device **10.** Overhanging balloon **14** is uninflated and dilator **16** extends past balloon **14.** It is noted that the relative sizes of sheath **12** and dilator **14** shown are for illustrative purposes as the diameter of dilator **14** may be relatively larger or smaller than shown in relation to the diameter of sheath **12,** although dilator **14** necessarily has a smaller diameter than sheath **12.** Although dilator **14** is shown to have a pointed end, dilator **14** may have a rounded or relatively flat end. Embodiments, as described herein, are designed and intended to puncture septum without use of a needle or other sharp instrument.

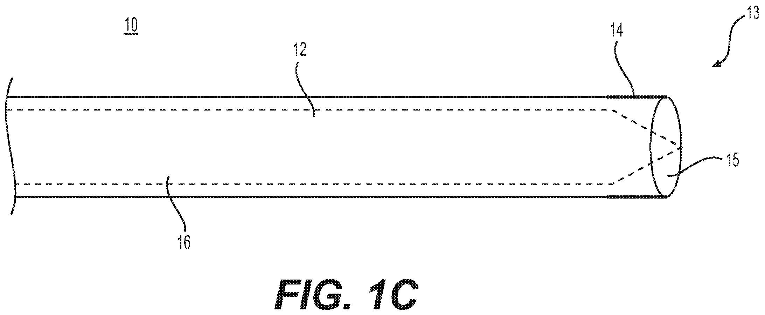

With reference now to FIG. 1C, dilator **16** is shown positioned within center lumen **15** of sheath **12.** Tip of dilator **16** is positioned within distal tip **13** of transseptal insertion device **10** sub-planar to end of transseptal insertion device **10.** The position shown is position dilator **16** may be in immediately prior to inflation of balloon **14.** It is noted that the relative sizes of catheter/sheath **12** and dilator **16** shown are for illustrative purposes as the diameter of dilator **16** may be relatively larger or smaller than shown in relation to the diameter of sheath **12.** Ordinarily, dilator **16** has smaller diameter or gauge then catheter/sheath **12,** although fit of dilator **16** in catheter/sheath **12** is preferably snug enough so that dilator **16** does not move (laterally or axially) relative to position or "wobble" within transseptal insertion device **10.** catheter **18** necessarily has a smaller diameter than sheath **12.** In embodiments, sheath **12** material may be sufficiently malleable to enable larger diameter dilators **16,** and other larger diameter devices, to be passed through sheath **12.** In such embodiments, the sheath **12** will stretch to accommodate the larger diameter dilator **16** or other device.

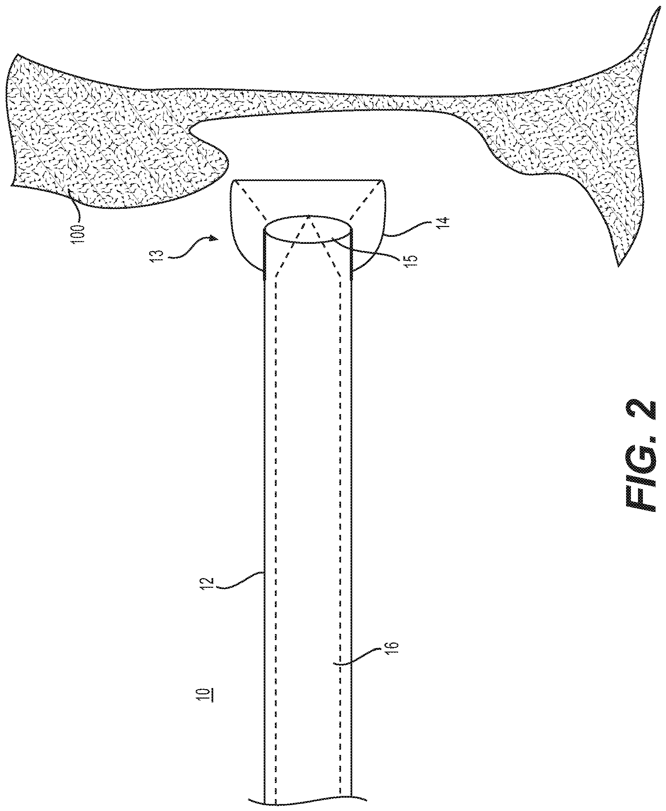

With reference now to FIG. 2, shown is distal end of an embodiment of transseptal insertion device **10** in which overhanging balloon **14** is inflated. Dilator **16** is shown positioned within center lumen **15** of sheath **12** with tip of dilator **16** positioned at distal tip **13** of transseptal insertion device **10** and sub-planar to overhanging balloon **14.** The plane that is referred to here is the plane X-X, perpendicular to the axis of transseptal insertion device **10** and dilator **16,** formed by the end of overhanging balloon **14.** Hence, dilator **16** remains sub-planar to overhanging balloon **14** until operator intends balloon **14** to be deflated and dilator **16** to tent and puncture interatrial septum 100. As noted above, balloon **14** preferably extends completely around circumference of tip **13** of transseptal insertion device **10.** Accordingly, FIG. 3 only illustrates cross-section of inflated balloon **14.**

With reference now to FIG. 3, shown is a perspective, cross-sectional view of distal end an embodiment of transseptal insertion device **10** in which overhanging balloon **14** is inflated. As shown, inflated overhanging balloon **14** preferably extends around entire circumference of sheath **12** (and, therefore, device **10**). Shown situated within lumen **15** of sheath **12** is tip of dilator **16.** Tip of dilator **16** is positioned within tip **13** of transseptal insertion device **10,** as it would be prior to being extended past tip **13** and puncturing an interatrial cardiac septum.

With reference now to FIG. 4, shown is shown is distal end of an embodiment of transseptal insertion device **10** with dilator **16** advanced forward in order to tent the interatrial septum **100.** Dilator **16** is shown extending through center lumen **15** of sheath **12** and past overhanging balloon **14.** Since dilator **16** is no longer sub-planar to overhanging balloon **14** and has moved past overhanging balloon **14,** dilator seal (not shown) has moved and unsealed balloon **14,** causing it to begin to deflate. Extended as such, and pressed against interatrial septum **100,** dilator **16** tents the interatrial septum **100** away from transseptal insertion device **10.**

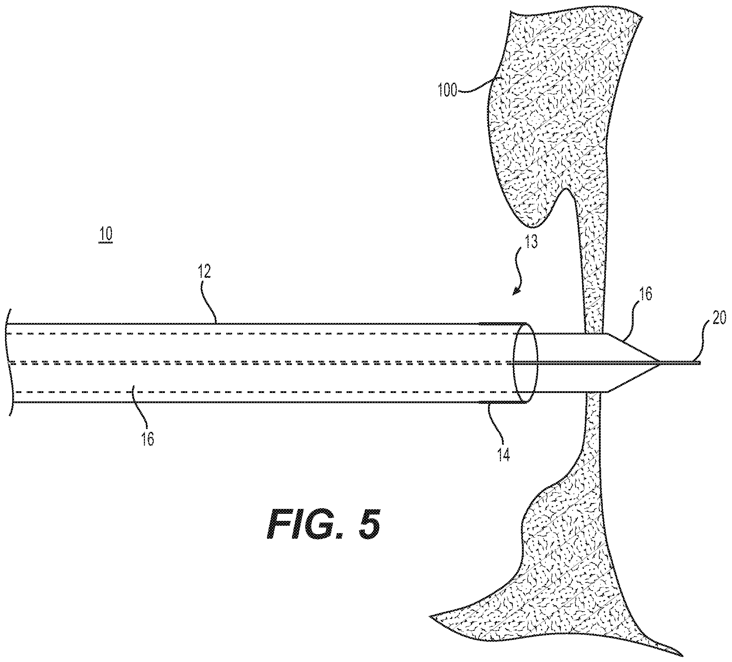

With reference now to FIG. 5, shown is shown is distal end of an embodiment of transseptal insertion device **10** with dilator **16** advanced forward through interatrial septum **100,** after puncturing septal wall (*e.g.*, through application of energy through dilator **16** as described herein) and transseptal wire or wire rail **20** extending through dilator **16** and into left atrium chamber **110.** Wire rail **20** may sit in lumen **19** of dilator **16.** Dilator **16** may be used as a conduit to advance the wire rail **20** into the left atrium.

Wire rail **20** may act as a guide for devices to enter the left atrium through the puncture in the septal wall made by transseptal insertion device **10.** For example, wire rail may guide transseptal insertion device **10** or other catheters in the left atrium. In this manner, catheters may be advanced safely into the left atrium over or guided by wire rail **20.** In an embodiment, wire rail **20** may be energized (*e.g*., to ablate or puncture the septum with energy delivered from source at proximal end of transseptal insertion device **10**).

With continued reference to FIG. 5, dilator **16** preferably defines and includes an opening or lumen **19** extending through its tip and through which transseptal wire **20** extends. With dilator **16** extended as shown and tenting interatrial septum, septum may be punctured by energy delivered through cap or electrode at tip of dilator **16** (see below) and transseptal wire rail **20** extended through opening in tip of dilator **16** and through puncture made in interatrial septum by dilator **16** cap.

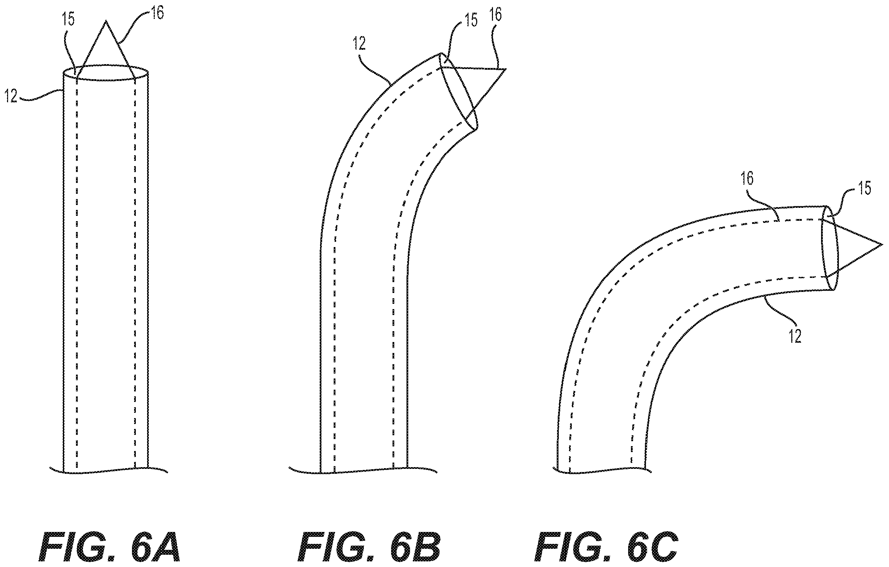

With reference to FIGS. 6A-6C, shown are different views of an embodiment of transseptal insertion device **10** with a flexible sheath **12** flexed or angulated at different angles. Transseptal insertion device **10** may be flexed or angulated depending on the anatomy of the atria using fixed angled dilators **16** that are inserted into lumen **15** of sheath **12,** causing sheath **12** to flex. Such fixed angled dilators **16** may be, *e.g*., any angle from 0-270°. Alternatively, sheath **12** and dilator **16** may be both flexible (preferably, needle and catheter inserted through such flexible sheath **12** are flexible or malleable, at least in part) and transseptal insertion device **10** may be flexed or angulated, thereby flexing or angulating sheath **12** and dilator **16,** using, *e.g*., a handle or wire (not shown) connected to tip **13** of device **10.** Handle and/or wire may also be used to turn or flex or move tip **13** of transseptal insertion device **10,** *e.g*., moving tip **13** of sheath "up" or "down" or "left" or "right" or angulating tip **13** relative to axis of sheath **12** as shown.

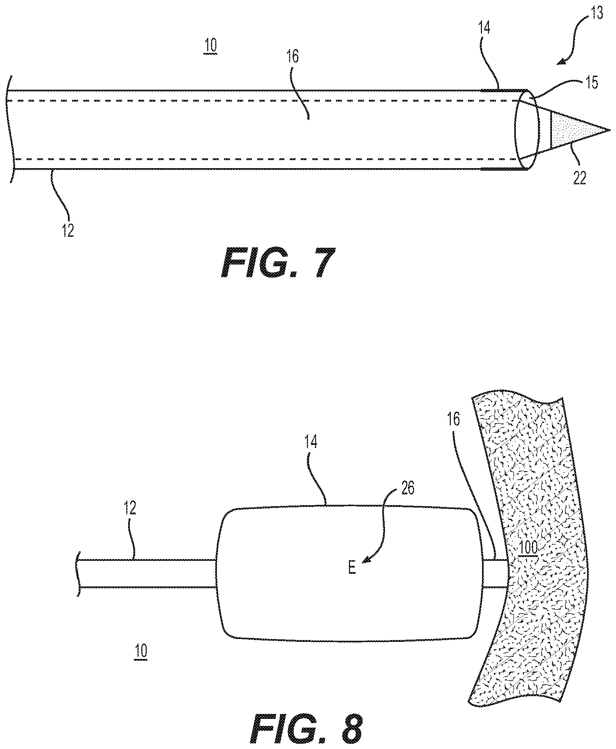

With reference now to FIG. 7, shown is an embodiment of transseptal insertion device **10** with radiofrequency energy capability. Transseptal insertion device **10** shown includes sheath **12,** overhanging balloon **14,** and dilator **16.** Dilator **16** may include cap or crown **22,** on distal end as shown, with RF energy capability or capable of delivering RF energy. Alternatively, cap or crown may include or be an RF electrode. Dilator **16** may be connected, *e.g*., on proximate end (not shown) to a radiofrequency (RF) energy source (not shown) at, *e.g*., external hub, that provides RF energy to cap or crown **22.** The RF energy may be delivered through dilator **16.** So equipped with cap or crown **22,** dilator **16** may tent interaxial septum and create puncture of interaxial septum through delivery of RF energy. In this embodiment, the use of a sharp needle may be avoided.

As described above, embodiments may be capable of delivering other energy, such as thermal, laser, sonic, or electrical energy for the purposes of puncturing the septum. Such embodiments may be constructed in a similar manner, with dilator or needle including cap or crown at a distal end capable of delivering thermal, laser, sonic, or electrical energy, and such energy may be delivered through dilator or needle connected, *e.g*., on proximate end (not shown) to a thermal, laser, sonic, or electrical energy source (not shown) at, *e.g.*, external hub. So connected, dilator or needle may use thermal, laser, ultrasound, or electrical energy to puncture interaxial septum, avoiding the need for a sharp needle.

A significant challenge for operators of transseptal devices today is the difficulty in determining how posterior (towards the back of a patient) the transseptal device is located. The left atrium is on the posterior side of the heart. It is, therefore, often critical to be able to determine how posterior is the distal tip **13** of transseptal insertion device **10** in order to successfully locate the interatrial septum. Generally tracking the location of the distal end of transseptal insertion device **10** is critical to safe operation. With reference now to FIG. 8, shown is distal end of an embodiment of transseptal insertion device **10** with inflated overhanging balloon **14.** Balloon **14** shown is an embodiment with one or more markers **24.** Marker **24** may be, *e.g*., a radiopaque and/or echogenic marker **24.** As a radiopaque or echogenic marker, marker **24** will be visible on scanners used by those performing cardiac catheterizations. The markers **24** may be in the form of letters, such as an E or a C. Marker **24** enables the appropriate positioning of balloon **14** and catheter **18** in the 3-dimensional space (*e.g.,* of the atrium) using imaging to view the marker **24** and, therefore, the position of balloon **14.**

Specifically, in operation, the less posterior distal tip **13** is positioned, the more of the E (or C) will be shown. As operator of transseptal insertion device **10** turns or rotates distal tip **13** toward posterior of patient, less of the arms of the E will be seen. In a preferred embodiment, when only the vertical portion of the E is visible (*i.e.*, appearing as an I) distal tip **13** will be rotated to its maximum posterior position. Consequently

With continuing reference to FIG. 8, balloon **14** is shown as inflated. However, distal end of dilator **16** is shown extruding or extending distally from balloon **14,** past plane formed by distal end of inflated balloon **14.** According, dilator **16** has been moved into the tenting and puncturing position, adjacent to interaxial septum, dilator seal (not shown) has exited or soon will exit sheath **12,** balloon **14** is deflating or will soon deflate, and puncture of the interaxial septum is imminent.

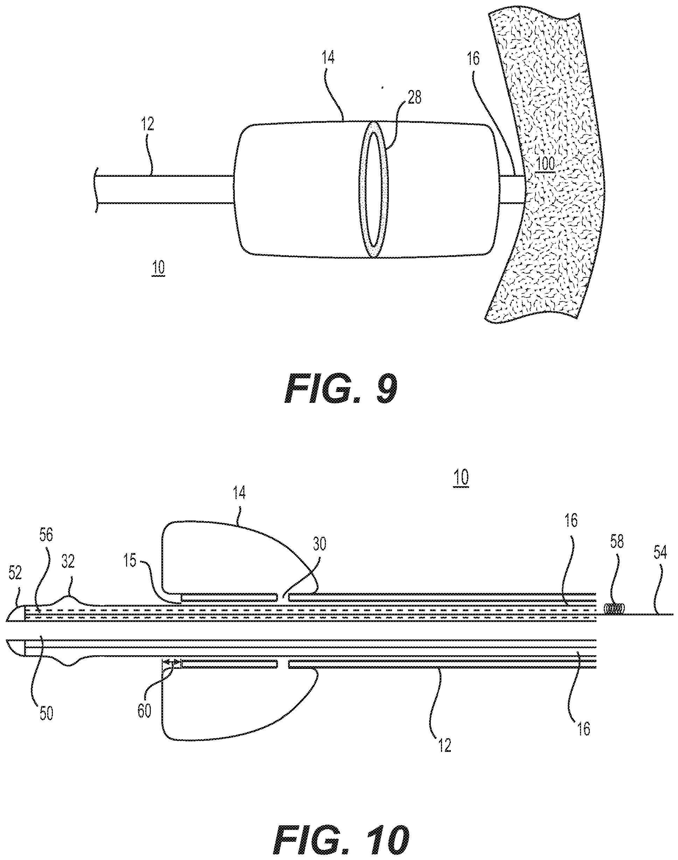

With reference now to FIG. 9, shown is another embodiment of overhanging balloon **14** which may be deployed in embodiments of transseptal insertion device **10.** Overhanging balloon **14** may include ring or band **28** around a portion of balloon **14.** Ring or band **28** may serve as a marker, similar to markers **24** shown in FIG. 8. Hence, ring **28** may be radiopaque or echogenic and may be view by scanning devices used for visualization in cardiac catheterizations (*e.g*., fluoroscopic imaging devices). Similar to the letter E or C, the view of the ring **28** changes as the distal tip **13** of transseptal insertion device **10** moves more posterior. When in a least posterior position, ring **28** may appear as just a line or band positioned across axis of transseptal insertion device **10.** When device **10** is rotated so that distal tip **13** is significantly closer to the posterior, ring **28** may appear as a full "flat" circle or ring. In FIG. 8, distal tip **13** is partially rotated so that ring **28** is partially visible.

With reference to both FIGS. 8 and 9, the marker **24** and ring **28** are described and shown as located on balloon **14.** In embodiments, marker **24** and/or ring **28** may also be located on sheath **12** and/or dilator **16.** So located, marker **24** and/or ring **28** would operate in effectively the same manner as described above (*i.e.,* the arms of the E would disappear as the distal end was moved more to the posterior and the ring would become more visible). Markers **24** and/or rings **28** may be placed on all three of balloon **14,** sheath **12,** and dilator **16,** or a combination thereof.

With reference now to FIG. 10, shown is distal end of an embodiment of transseptal insertion device **10** in accordance with the present claimed invention that includes dilator **16** with electrode tip. Shaft of dilator **16** defines and contains a center lumen **50.** Lumen **50** may be defined in the range of, but not limited to, .020"-.040". 1 inch = 2.54 cm. Dilator **16** may be made from a polymer material (*e.g*., HDPE, LDPE, PTFE, or combination thereof). Dilator shaft **16** shown includes a distal electrode tip **52.** Electrode tip **52** may be comprise a metallic alloy (*e.g*., PtIr, Au, or combination thereof). In preferred embodiments, the size and shape of electrode tip **52** is selected to be sufficient to generate a plasma for *in vivo* ablation of tissue in an applied power range of, but not limited to, 20-30W. Electrical conductor **54** extends from electrode tip **52** to the proximal end (not shown) of the dilator **16.** Electrical conductor **54** may run axially through an additional lumen **56** defined by and contained in dilator shaft **16.** Electrical conductor **54** may contain a coil feature **58** to accommodate lengthening during bending or flexing of dilator **16.**

Dilator **16** also includes a distal dilator seal **32** for occlusion of sheath shaft **12** center lumen **15.** Dilator seal **32** may be a ring that extends around entire circumference of dilator **16.** Dilator seal **32** may be in the range of, but not limited to, 0.000"-0.005" larger in diameter than center lumen **15.** 1 inch = 2.54 cm. Distal seal **32** occludes lumen **15,** forming a liquid and gas tight seal so that balloon **14** remains inflated, until distal seal **32** exits lumen **15.** Attached to distal end of sheath **12** is contains overhanging balloon **14.** Overhanging balloon **14** may be made from a polymer material (*e.g*., PET, Nylon, Polyurethane, Polyamide, or combination thereof). Overhanging balloon **14** may be in the range of, but not limited to, 5-20mm in diameter and 20-30mm in length. Overhanging balloon **14** may be inflated via injection of fluid (or gas) from the proximal end of sheath **12** center lumen **15** while distal dilator seal **32** occludes center lumen **15** distal to inflation ports **30** in sheath **12.** Inflation ports **30** are preferably defined flush to surface of sheath **12** and communicate with lumen **15** (*e.g.,* inflation ports may simply be holes defined in sheath that connect lumen to exterior of sheath). Balloon **14** is preferably connected to sheath **12** so that inflation ports **30** communicate with interior of balloon **14** and provide pathway for inflation fluid or gas to enter and inflate balloon **14** (and exit and deflate balloon **14**). During the proper functioning or operation of transseptal insertion device **10** for puncturing the interatrial septum, balloon **14** is deflated when dilator **16** moves out of lumen **15** and dilator seal **32** moves distal and outside of sheath **12.** However, deflation of overhanging balloon **14** may occur either via positioning of dilator seal **32** proximal to inflation ports **30** or distal and outside of sheath **12.** Overhanging balloon **14** is of form such balloon **14** overhangs or extends from distal end **13** of sheath **12.** Overhang or extension **60** may be in the range of, but not limited to, 0.0mm-5.0mm. The end of the overhang or extension **60** is the plane to which dilator **16** remains sub-planar until moving to tent and puncture the interatrial septum.

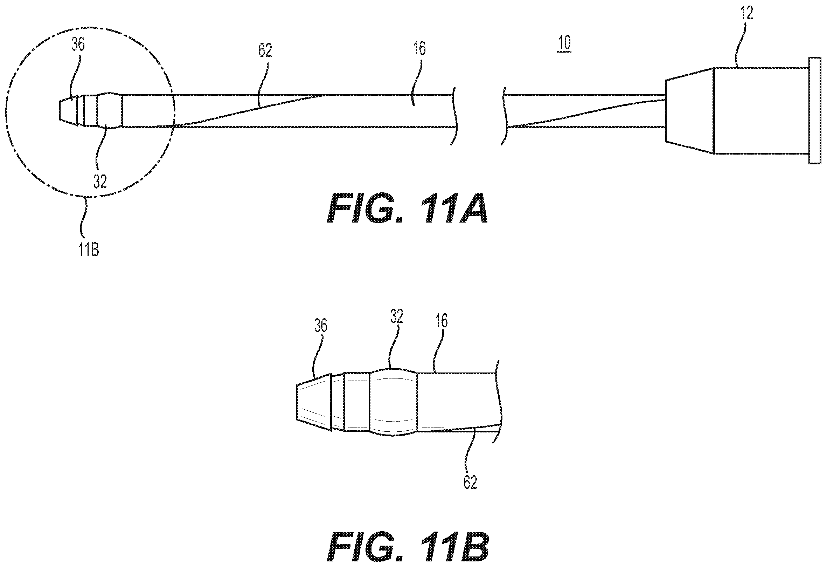

With reference now to FIGS. 11A and 11B, shown is a distal end of an embodiment of transseptal insertion device **10** with RF capability, with dilator **16** extended out from sheath **12** shaft. Dilator **16** includes a RF cap or tip **36** that may deliver RF energy for interatrial septum ablation purposes, as described above. RF cap **36** may be connected to RF energy source (not shown) at proximal end (not shown) of transseptal insertion device **10** with conductor **62.** Conductor **62** may wrap around shaft of dilator **16** as shown. Alternatively, conductor **62** may be extend through a lumen (not shown) of dilator **16** (*e.g*., such as lumen **56** shown in FIG. 18). Dilator **16** may include distal dilator seal **32** for sealing center lumen **15** (not shown) of sheath **12** and inflation ports **30** (not shown).

Variations of the above embodiments are within the scope of the invention. For example, the dilator shaft may have a preformed shape other than straight. The dilator shaft may contain a deflection apparatus. The electrode tip may be the distal dilator seal. The electrical conductor may wrap around the center lumen. Sheath or balloon shaft may contain a deflection apparatus.

Embodiments of transseptal insertion device **10** can successfully assist surgeons in carrying out at least one of the following techniques: visualization and stabilization of the intra atrial septum; visualization and stabilization of the fossa ovalis; and, guidance for transseptal puncture and across septum into safe zone of left atrium (away from structures such as aorta).

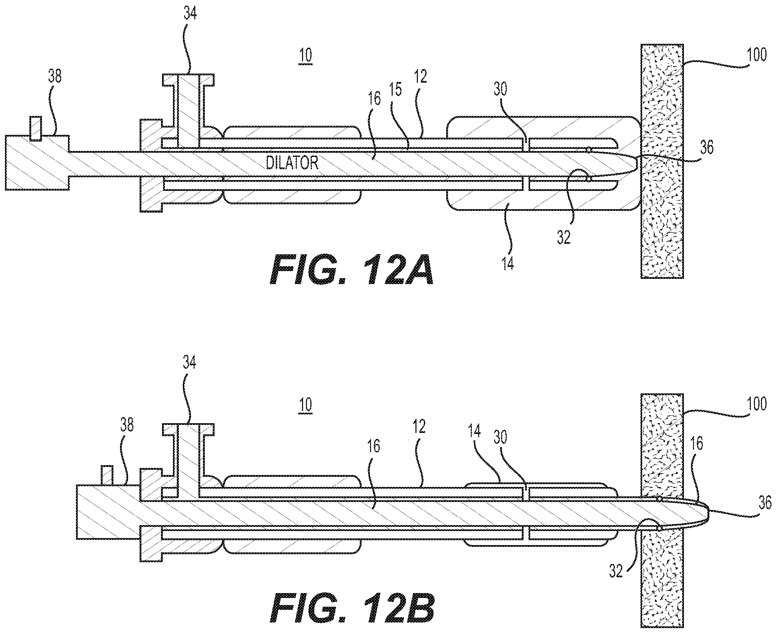

With reference now to FIGS. 12A-12B, shown is another embodiment of transseptal insertion device **10** that inflates overhanging balloon **14** using gas or fluid via sheath **12.** Embodiments include no separate port or hypotube for inflation or deflation of balloon **14.** Transseptal insertion device **10** may include inflation ports **30** in sheath **12** and dilator seal **32** on dilator **16.** Gas (*e.g*., air) or liquid is input into sheath **12** through inlet or port **34.** Gas or liquid exits sheath **12** through inflation ports **30,** inflating balloon **14** until fully inflated or inflated as much as desired. When dilator seal **32** is covered by inflated, overhanging sheath balloon **14,** as shown in FIG. 12A, dilator seal **32** is closed and balloon **14** remains inflated. As dilator **16** is advanced beyond overhanging balloon **14,** as shown in FIG. 12B, dilator seal **32** moves beyond the seal zone, dilator seal **32** is opened or unsealed, resulting in balloon **14** automatically and rapidly releasing inflation gas or fluid and deflating. The inflation gas or liquid exits the balloon **14** and the lumen **15** as noted above and is absorbed by the body and bloodstream. As such, inflation gas or liquid is inert and non-harmful. Inflation gas or fluid may include contrast agents enabling easier detection of inflation and deflation using imaging.

Embodiments of transseptal insertion device **10** include ablation tip **36,** *e.g*., radiofrequency (RF) ablation tip **36** (similar to crown or cap **22**) that may be used to deliver RF or other energy to ablate interaxial septum (septal wall) in order to puncture and create opening in septum. Transseptal insertion device **10** may include energy source at proximal end to deliver RF or other energy through dilator to ablation tip **36.** Energy source may be, *e.g.,* RF ablation connector on external hub **38.** Ablation connector on external hub **38** may be connected to proximal end of dilator **16,** as shown.

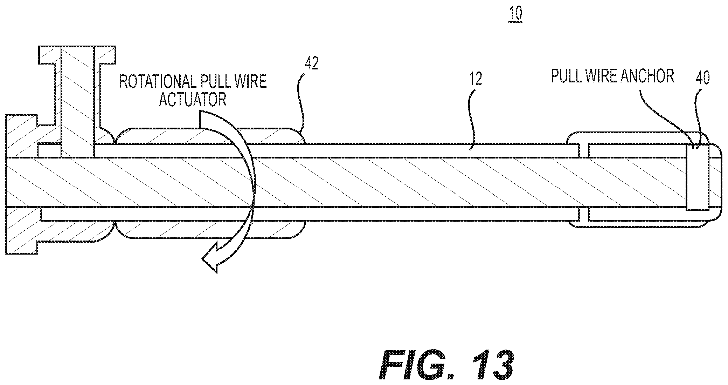

With reference now to FIG. 13, shown is an embodiment of transseptal insertion device 10 that includes a mechanical deflection mechanism. Mechanical deflection mechanism may enable distal end of sheath **12** to be deflected or angulated to various angles with respect to axis of transseptal insertion device **10.** Mechanical deflection mechanism may include a pull wire anchor **40** affixed to distal end of sheath **12** and pull wire actuator **42** connected to pull wire anchor **40** with pull wire (not shown). Rotation of pull wire actuator **42,** as shown, may exert force on pull wire anchor **40** that deflects or angulates distal end of sheath **12.** Pull wire actuator **42** may be rotated by handle connected thereto (not shown). Deflection or angulation of distal end of sheath **12** may enable better intersection (*e.g*., more perpendicular, flush) with interaxial septum and, therefore, better puncture and insertion by transseptal insertion device **10.**

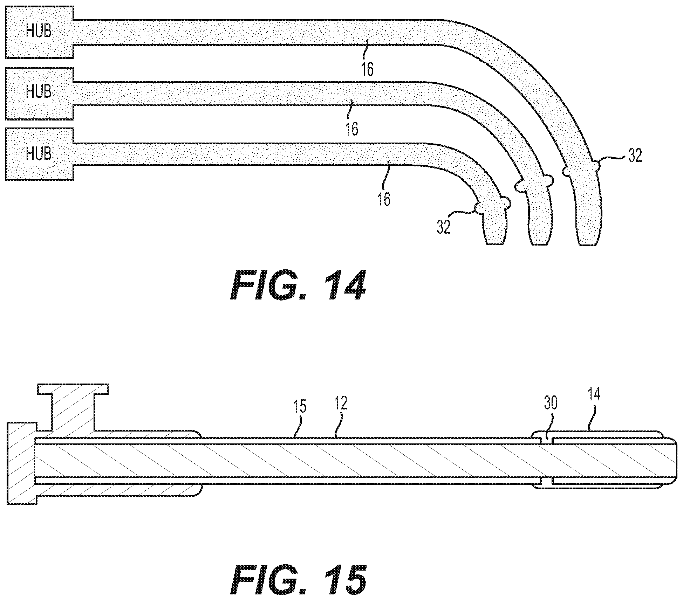

With reference now to FIG. 14, shown are three (3) embodiments of curved dilators **16,** each with a different curve profile (*i.e.,* different angle of deflection or curve). Curved dilators **16** may be used in embodiments of transseptal insertion device **10** with flexible or malleable sheath **12.** Such a flexible or malleable sheath **12** may be referred to as a steerable sheath **12** as it is 'steered" by curved dilator **16** inserted in sheath **12.**

With reference now to FIG. 15, shown is an embodiment of transseptal insertion device 10 that includes a steerable sheath **12.** In embodiment shown, distal end of sheath **12** is flexible or malleable so that sheath **12** may bend or angulate, *i.e.,* be steered, when, for example, curved dilator **16** is inserted. Proximal body of sheath **12** may be stiffened so that curved dilator **16** may be more easily pushed through sheath **12** when inserted therein.

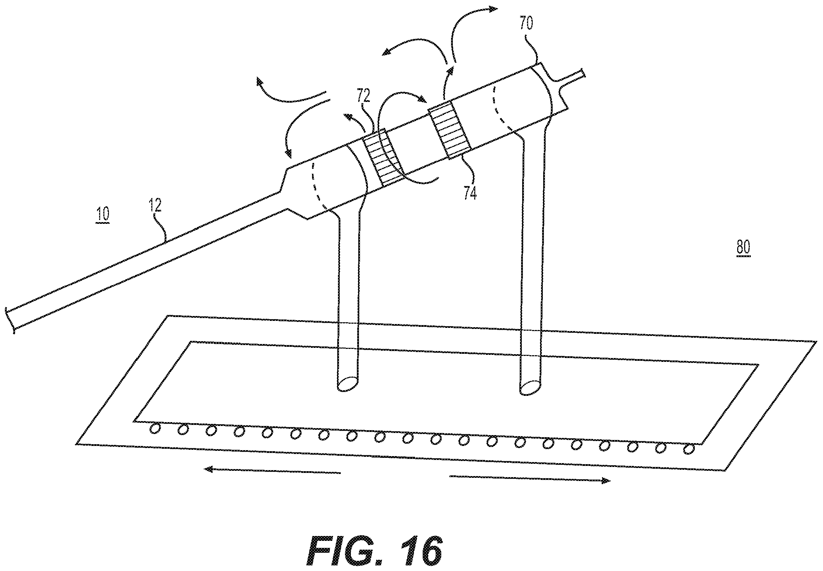

Embodiments of transseptal insertion device **10** may include a stabilizer in which the exterior of the catheter would be placed and which allows for very precise movements of the catheter. With reference now to FIG. 16, shown is an embodiment of transseptal insertion device/catheter **10** with an external stabilizer **80.** Stabilizer **80** keeps proximal end of transseptal insertion device **10** stable while allowing movement of transseptal insertion device **10** towards the distal and proximal ends of device **10,** rotational/torqueing movement of proximal end of device **10,** and manipulation of dials or other controls of device **10.** In effect, stabilizer **80** substantially prevents unwanted movement of the transseptal insertion device **10** and, importantly, distal end of sheath **12,** balloon **14,** and dilator **16.**

Stabilizer **80** includes connecting rods or arms **82** that connect stabilizer **80** to handle **70** at proximal end of transseptal insertion device **10.** Connecting arms **82** are attached to stabilizer platform **84.** Connecting arms **82** preferably hold the handle **70** securely and tightly, while permitting desired rotational movements and control manipulation. Stabilizer platform **84** is moveably attached to stabilizer base **86** so that stabilizer platform **84,** and hence handle **70** and transseptal insertion device **10,** may be slid forwards and backwards along axis of transseptal insertion device **10** towards and away from insertion point in patient (typically femoral vein at the groin of patient). Stabilizer base **86** is typically secured to a flat, stable surface, such as a table, or the leg of the patient. Configured as such, stabilizer **86** prevents unwanted vertical, rotational, or other movement of transseptal insertion device **10** and its handle **70,** keeping transseptal insertion device **10** and its handle **70** stable while permitting precise manipulation of handle **70** and its controls.

Since many modifications, variations, and changes in detail can be made to the described preferred embodiments of the invention, it is intended that all matters in the foregoing description and shown in the accompanying drawings be interpreted as illustrative and not in a limiting sense. Consequently, the scope of the invention should be determined by the appended claims.

## Claims

1. A transseptal insertion device (10) which is suitable for facilitating precise and safe transseptal puncture of a cardiac interatrial septum (100), comprising:
a sheath (12) that defines at least one lumen (15) therein and has a distal end that is closest to the cardiac interatrial septum of a patient when the transseptal insertion device is in use and a proximal end that is external to the patient;
a balloon (14) that is connected to the distal end of the sheath (12), wherein the balloon, when inflated and the transseptal insertion device (10) is in use, overhangs and extends past the distal end of the sheath (12), preventing accidental puncturing of the cardiac interatrial septum (100) and stabilizing the transseptal insertion device (10) against fossa ovalis of the cardiac interatrial septum; and
a dilator (16) that is positioned within the at least one lumen (15) when the transseptal insertion device (10) is in use, wherein the dilator (16) has a distal end
**characterised in that** the dilator (16)
is designed to and is capable of precisely puncturing the cardiac interatrial septum (100) without the use of a needle or other sharp instrument,
and **in that** the dilator (16) includes a dilator seal (32) that is greater in diameter than the lumen (15) and provides a water-tight seal of the lumen while the dilator seal remains within the lumen,
and wherein the sheath further comprises inflation ports communicatively coupling the lumen to the balloon so that inflation liquid may be passed through lumen into the balloon, inflating the balloon.

2. The transseptal insertion device of claim 1 wherein the balloon portion that overhangs and extends past the distal end of the sheath (12) is the overhanging portion and the dilator (16) is designed to remain sub-planar to the overhanging portion of the balloon until the dilator (16) is extended to puncture the cardiac interatrial septum (100).

3. The transseptal insertion device of claim 1 wherein the sheath (12) is made from malleable material capable of accommodating larger diameter devices within the lumen (15).

4. The transseptal insertion device of claim 1 wherein the dilator seal (32) is located on the distal end of the dilator (16) and moving the distal end of the dilator out of the lumen (15) so that the dilator seal is external to the distal end of the sheath (12) un-seals the lumen and causes the inflation liquid to flow out of the balloon (14) and through the inflation ports (30), deflating the balloon.

5. The transseptal insertion device of claim 1 further comprising an energy source external to the proximal end of the sheath (12) and operatively connected to the distal end of the dilator (16) to deliver energy to the distal end of the dilator, in particular wherein the dilator (16) comprises a cap (22,36) on the distal end of the dilator capable of applying the delivered energy to the cardiac interatrial septum (100).

6. The transseptal insertion device of claim 5 wherein the dilator (16) is designed to and is capable of precisely puncturing the cardiac interatrial septum (100) using the energy delivered to the distal end of the dilator, in particular wherein the energy source is a radio-frequency (RF) energy source and the energy delivered to the distal end of the dilator is RF energy.

7. The transseptal insertion device of claim 1 further comprising an external stabilizer (80) connected to the proximal end of the sheath (12) that stabilizes the transseptal insertion device (10) and substantially prevents unwanted movement of the transseptal insertion device.

8. The transseptal insertion device of claim 1 wherein the balloon (14) includes one or more radiopaque or echogenic markers (24), in particular wherein the one or more markers include a marker in the shape of an E or a ring-shaped marker.

9. The transseptal insertion device according to claim 1,
wherein the balloon (14) is positioned over the inflation ports (30) of the sheath (12) so that inflation fluid may exit the sheath and enter the balloon, inflating the balloon, and exit the balloon and enter the sheath, deflating the balloon;
and wherein the dilator (16) includes said dilator seal (32) at the distal end that occludes the lumen (15) when the dilator (16) is sub-planar to the balloon overhang so that the balloon (14) remains inflated after inflation fluid is passed through the lumen (15) into the balloon (14) and automatically deflates when the distal end of the dilator (16) is extended sufficiently past the distal end of the sheath (12) so that the dilator seal does not occlude the lumen.

10. The transseptal insertion device of claim 9 further comprising an RF energy source external to the proximal end of the sheath (12) and operatively connected to the distal end of the dilator (16) to deliver RF energy to the distal end of the dilator, in particular wherein the dilator comprises a cap (22,36) on the distal end of the dilator (16) capable of applying the delivered RF energy to the cardiac interatrial septum (100) and precisely puncturing the cardiac interatrial septum using the delivered RF energy.

## Patentansprüche

1. Vorrichtung (10) zur transseptalen Einführung, die zur Erleichterung der präzisen und sicheren transseptalen Punktion eines interatrialen Septums (100) des Herzens geeignet ist, umfassend:
eine Hülse (12), die mindestens ein Lumen (15) darin definiert und ein distales Ende, das sich am nächsten zu dem interatrialen Septum des Herzens eines Patienten befindet, wenn die Vorrichtung zur transseptalen Einführung in Gebrauch ist, und ein proximales Ende aufweist, das sich außerhalb des Patienten befindet;
einen Ballon (14), der mit dem distalen Ende der Hülse (12) verbunden ist, wobei der Ballon, wenn er befüllt ist und die Vorrichtung (10) zur transseptalen Einführung in Gebrauch ist, überhängt und sich über das distale Ende der Hülse (12) hinaus erstreckt, wodurch versehentliche Punktion des interatrialen Septums (100) des Herzens verhindert wird und die Vorrichtung (10) zur transseptalen Einführung gegen die Fossa ovalis des interatrialen Septums des Herzens stabilisiert wird; und
einen Dilator (16), der innerhalb des mindestens einen Lumens (15) positioniert ist, wenn die Vorrichtung (10) zur transseptalen Einführung in Gebrauch ist, wobei der Dilator (16) ein distales Ende aufweist,
**dadurch gekennzeichnet, dass** der Dilator (16) konzipiert und in der Lage ist, das interatriale Septum (100) des Herzens präzise zu durchstechen, ohne dass eine Nadel oder sonstiges scharfes Instrument verwendet wird,
und dass der Dilator (16) eine Dilatordichtung (32) einschließt, die einen größeren Durchmesser als das Lumen (15) aufweist und eine wasserdichte Dichtung des Lumens bereitstellt, während die Dilatordichtung innerhalb des Lumens bleibt,
und wobei die Hülse des Weiteren Befüllungsports umfasst, die das Lumen kommunikativ an den Ballon koppeln, so dass Befüllungsflüssigkeit durch das Lumen hindurch in den Ballon gegeben werden kann, wodurch der Ballon befüllt wird.

2. Vorrichtung zur transseptalen Einführung nach Anspruch 1, wobei der Ballonanteil, der überhängt und sich über das distale Ende der Hülse (12) hinaus erstreckt, der überhängende Anteil ist und der Dilator (16) konzipiert ist, um subplanar zu dem überhängenden Anteil des Ballons zu bleiben, bis der Dilator (16) ausgefahren wird, um das interatriale Septum (100) des Herzens zu durchstechen.

3. Vorrichtung zur transseptalen Einführung nach Anspruch 1, wobei die Hülse (12) aus einem formbaren Material gefertigt ist, das in der Lage ist, Vorrichtungen mit größerem Durchmesser innerhalb des Lumens (15) unterzubringen.

4. Vorrichtung zur transseptalen Einführung nach Anspruch 1, wobei die Dilatordichtung (32) sich auf dem distalen Ende des Dilators (16) befindet, und wobei Bewegung des distalen Endes des Dilators aus dem Lumen (15) heraus, so dass die Dilatordichtung außerhalb des distalen Endes der Hülse (12) liegt, die Dichtung des Lumens aufhebt und bewirkt, dass die Befüllungsflüssigkeit aus dem Ballon (14) heraus und durch die Befüllungsports (30) fließt, wodurch der Ballon entleert wird.

5. Vorrichtung zur transseptalen Einführung nach Anspruch 1, des Weiteren umfassend eine Energiequelle außerhalb des proximalen Endes der Hülse (12) und funktionell verbunden mit dem distalen Ende des Dilators (16), um dem distalen Ende des Dilators (16) Energie zuzuführen, wobei insbesondere der Dilator (16) eine Kappe (22, 36) auf dem distalen Ende des Dilators umfasst, die in der Lage ist, die zugeführte Energie auf das interatriale Septum (100) des Herzens anzuwenden.

6. Vorrichtung zur transseptalen Einführung nach Anspruch 5, wobei der Dilator (16) konzipiert und in der Lage ist, das interatriale Septum (100) des Herzens unter Verwendung der dem distalen Ende des Dilators zugeführten Energie präzise zu durchstechen, wobei insbesondere die Energiequelle eine Hochfrequenz- (HF)-Energiequelle ist und die dem distalen Ende des Dilators zugeführte Energie HF-Energie ist.

7. Vorrichtung zur transseptalen Einführung nach Anspruch 1, des Weiteren umfassend einen externen Stabilisator (80), der mit dem proximalen Ende der Hülse (12) verbunden ist und die Vorrichtung (10) zur transseptalen Einführung stabilisiert und im Wesentlichen ungewollte Bewegung der Vorrichtung zur transseptalen Einführung verhindert.

8. Vorrichtung zur transseptalen Einführung nach Anspruch 1, wobei der Ballon (14) einen oder mehrere röntgendichte oder echogene Marker (24) einschließt, wobei insbesondere der eine oder die mehreren Marker einen Marker in Form von einem E oder einen ringförmigen Marker einschließt/einschließen.

9. Vorrichtung zur transseptalen Einführung nach Anspruch 1,
wobei der Ballon (14) über den Befüllungsports (30) der Hülse (12) positioniert ist, so dass Befüllungsfluid aus der Hülse austreten und in den Ballon eintreten kann, wodurch der Ballon befüllt wird, und aus dem Ballon austreten und in die Hülse eintreten kann, wodurch der Ballon entleert wird; und
wobei der Dilator (16) die Dilatordichtung (32) an dem distalen Ende einschließt, die das Lumen (15) okkludiert, wenn der Dilator (16) subplanar zu dem Ballonüberhang ist, so dass der Ballon (14) befüllt bleibt, nachdem Befüllungsfluid durch das Lumen (15) hindurch in den Ballon (14) gegeben wurde, und sich automatisch entleert, wenn das distale Ende des Dilators (16) ausreichend über das distale Ende der Hülse (12) hinaus ausgefahren wird, so dass die Dilatordichtung das Lumen nicht okkludiert.

10. Vorrichtung zur transseptalen Einführung nach Anspruch 9, des Weiteren umfassend eine HF-Energiequelle außerhalb des proximalen Endes der Hülse (12) und funktionell verbunden mit dem distalen Ende des Dilators (16), um dem distalen Ende des Dilators HF-Energie zuzuführen, wobei insbesondere der Dilator eine Kappe (22, 36) auf dem distalen Ende des Dilators (16) umfasst, die in der Lage ist, die zugeführte HF-Energie auf das interatriale Septum (100) des Herzens anzuwenden und das interatriale Septum des Herzens unter Verwendung der zugeführten HF-Energie zu durchstechen.

## Revendications

1. Dispositif transseptal d'insertion (10) qui est approprié pour faciliter une perforation transseptale précise et sûre d'un septum interauriculaire cardiaque (100), comprenant :
une gaine (12) qui définit au moins une lumière (15) en son sein et présente une extrémité distale qui est la plus proche du septum interauriculaire cardiaque d'un patient lorsque le dispositif transseptal d'insertion est en cours d'utilisation et une extrémité proximale qui est extérieure au patient ;
un ballonnet (14) qui est relié à l'extrémité distale de la gaine (12), dans lequel le ballonnet, lorsqu'il est gonflé et que le dispositif transseptal d'insertion (10) est en cours d'utilisation, surplombe et s'étend au-delà de l'extrémité distale de la gaine (12), empêchant une perforation accidentelle du septum interauriculaire cardiaque (100) et stabilisant le dispositif transseptal d'insertion (10) contre la fosse ovale du septum interauriculaire cardiaque ; et
un dilatateur (16) qui est positionné à l'intérieur de l'au moins une lumière (15) lorsque le dispositif transseptal d'insertion (10) est en cours d'utilisation, dans lequel le dilatateur (16) présente une extrémité distale
**caractérisé en ce que** le dilatateur (16) est conçu pour et est susceptible de perforer avec précision le septum interauriculaire cardiaque (100) sans l'utilisation d'une aiguille ou d'un autre instrument pointu,
et **en ce que** le dilatateur (16) comprend un joint de dilatateur (32) dont le diamètre est supérieur à celui de la lumière (15) et qui assure l'étanchéité de la lumière pendant que le joint de dilatateur reste à l'intérieur de la lumière,
et dans lequel la gaine comprend en outre des orifices de gonflage accouplant en communication la lumière au ballonnet de sorte qu'un liquide de gonflage puisse être passé à travers la lumière dans le ballonnet, gonflant le ballonnet.

2. Dispositif transseptal d'insertion selon la revendication 1 dans lequel la partie de ballonnet qui surplombe et s'étend au-delà de l'extrémité distale de la gaine (12) est la partie en surplomb et le dilatateur (16) est conçu pour rester sous-planaire à la partie en surplomb du ballonnet jusqu'à ce que le dilatateur (16) soit étendu pour percer le septum interauriculaire cardiaque (100).

3. Dispositif transseptal d'insertion selon la revendication 1 dans lequel la gaine (12) est réalisée en matériau malléable susceptible d'accueillir des dispositifs de plus grand diamètre à l'intérieur de la lumière (15).

4. Dispositif transseptal d'insertion selon la revendication 1 dans lequel le joint de dilatateur (32) est situé sur l'extrémité distale du dilatateur (16) et le déplacement de l'extrémité distale du dilatateur hors de la lumière (15) de sorte que le joint de dilatateur soit externe à l'extrémité distale de la gaine (12) supprime l'étanchéité de la lumière et provoque l'écoulement du liquide de gonflage hors du ballonnet (14) et à travers les orifices de gonflage (30), dégonflant le ballonnet.

5. Dispositif transseptal d'insertion selon la revendication 1 comprenant en outre une source d'énergie externe à l'extrémité proximale de la gaine (12) et fonctionnellement connectée à l'extrémité distale du dilatateur (16) pour délivrer de l'énergie à l'extrémité distale du dilatateur, en particulier dans lequel le dilatateur (16) comprend un capuchon (22, 36) sur l'extrémité distale du dilatateur susceptible d'appliquer l'énergie délivrée au septum interauriculaire cardiaque (100).

6. Dispositif transseptal d'insertion selon la revendication 5 dans lequel le dilatateur (16) est conçu pour et est susceptible de perforer avec précision le septum interauriculaire cardiaque (100) à l'aide de l'énergie délivrée à l'extrémité distale du dilatateur, en particulier dans lequel la source d'énergie est une source d'énergie radiofréquence (RF) et l'énergie délivrée à l'extrémité distale du dilatateur est une énergie RF.

7. Dispositif transseptal d'insertion selon la revendication 1 comprenant en outre un stabilisateur externe (80) connecté à l'extrémité proximale de la gaine (12) qui stabilise le dispositif transseptal d'insertion (10) et empêche sensiblement un mouvement indésirable du dispositif transseptal d'insertion.

8. Dispositif transseptal d'insertion selon la revendication 1 dans lequel le ballonnet (14) comprend un ou plusieurs marqueurs radio-opaques ou échogènes (24), en particulier dans lequel les un ou plusieurs marqueurs comprennent un marqueur en forme de E ou un marqueur en forme d'anneau.

9. Dispositif transseptal d'insertion selon la revendication 1,
dans lequel le ballonnet (14) est positionné sur les orifices de gonflage (30) de la gaine (12) de sorte que le fluide de gonflage puisse sortir de la gaine et entrer dans le ballonnet, gonflant le ballonnet, et sortir du ballonnet et entrer dans la gaine, dégonflant le ballonnet ;
et dans lequel le dilatateur (16) comprend ledit joint de dilatateur (32) à l'extrémité distale qui obstrue la lumière (15) lorsque le dilatateur (16) est sous-planaire par rapport au surplomb du ballon de sorte que le ballon (14) reste gonflé après que le fluide de gonflage est passé à travers la lumière (15) dans le ballonnet (14) et se dégonfle automatiquement lorsque l'extrémité distale du dilatateur (16) est étendue suffisamment au-delà de l'extrémité distale de la gaine (12) de sorte que le joint de dilatateur n'obstrue pas la lumière.

10. Dispositif transseptal d'insertion selon la revendication 9 comprenant en outre une source d'énergie RF externe à l'extrémité proximale de la gaine (12) et connectée de manière opérationnelle à l'extrémité distale du dilatateur (16) pour délivrer une énergie RF à l'extrémité distale du dilatateur, en particulier dans lequel le dilatateur comprend un capuchon (22, 36) sur l'extrémité distale du dilatateur (16) susceptible d'appliquer l'énergie RF délivrée au septum interauriculaire cardiaque (100) et de perforer avec précision le septum interauriculaire cardiaque à l'aide de l'énergie RF délivrée.
